# EUROPEAN PATENT APPLICATION

(11) **EP 3 808 352 A1**
(43) Date of publication of application: **21.04.2021**
(21) Application number: 19819593.5
(22) Date of filing: 14.06.2019
(51) Int. Cl.: A61K 31/7032, A61P 27/06, A61P 27/02, A61P 27/12, A61P 17/02, A61P 9/10, A61P 3/10

(54) **USE OF SALIDROSIDE AND DERIVATIVE THEREOF IN PREPARATION OF INHIBITOR MEDICAMENT FOR DISEASES OF OPHTHALMIC FIBROSIS CAUSED BY ABNORMALITIES OF EXTRACELLULAR MATRIX PROTEINS**

(30) Priority: 15.06.2018 CN 201810620554
(71) Applicant: Shanghai 9th People's Hospital, Shanghai Jiaotong University School Of Medicine, Shanghai 200011 (CN)
(72) Inventor: GUO, Tao, Shanghai 200011 (CN); FAN, Xianqun, Shanghai 200011 (CN); FAN, Yuchen, Shanghai 200011 (CN); GUO, Li, Shanghai 200011 (CN); HOU, Iok-, Shanghai 200011 (US); WEI, Jiahong, Shanghai 200011 (CN)
(74) Representative: Icosa
(86) International application number: PCT/CN2019/091203
(87) International publication number: WO 2019/238107

(57) **Abstract**

The present invention provides use of salidroside and a derivative thereof in preparation of an inhibitor medicament for treating diseases, such as opthmalmic fibrosis, caused by abnormalities of extracellular matrix proteins. The present invention treats and prevents diseases associated with ocular fibrosis such as glaucoma, by inhibiting the expression of extracellular matrix proteins in the eye to prevent fibrosis. The present invention further provides use of an inhibitor medicament for treating and preventing diseases, such as opthmalmic fibrosis, caused by abnormalities of extracellular matrix proteins, the inhibitor medicament comprising an effective amount of salidroside and a derivative thereof as active ingredients.

## Description

### FIELD OF INVENTION

The invention relates to use of salidroside and derivatives thereof, specifically, to the application of salidroside and derivatives thereof in the preparation of inhibitors for treating ophthalmological diseases associated with fibrosis related to extracellular matrix protein abnormalities.

### BACKGROUND

The activation and stimulation of many cells and cytokines are a common feature in proliferative disorders in ophthalmology. Although the pathogeneses, treatment methods and prognoses of these abnormalities are different, the activation and stimulation of cells and cytokines cause abnormalities in extracellular matrix proteins, which lead to cell migration and proliferation, and great difficulties in clinical treatment and prognosis.

Primary open-angle glaucoma (POAG), scar formation after glaucoma filtration surgery, proliferative vitreoretinopathy, proliferative diabetic retinopathy, secondary cataract and other diseases are common ophthalmological disorders. Although the pathogeneses, treatment methods and prognoses are different, one common feature in these disorders is that many cells and cytokines participate in disease genesis and development. The activation and stimulation of pro-inflammatory factors in disease regions cause abnormalities of extracellular matrix proteins, leading to cell migration and proliferation, which bring great challenges to the clinical work of ophthalmologists and the prognosis of surgery.
1. In primary open-angle glaucoma, the pathological changes in the outflow pathway of the aqueous humor, especially changes in the trabecular meshwork will cause blockage of aqueous humor outflow and increased intraocular pressure. These changes are closely related to fibrosis of the trabecular meshwork and narrowing/closing of the meshwork openings, which are caused by abnormal accumulation of the trabecular meshwork extracellular matrix proteins, such as fibronectin (FN) and laminin (LA). In addition, some cytokines secreted by the trabecular meshwork, such as Transforming Growth Factor-beta 2 (TGF-beta 2), also affect the outflow of aqueous humor to various degrees. At present, the treatment of glaucoma is limited to reducing intraocular pressure in order to relieve symptoms. Ocular hypotensive drugs commonly used in clinic can be divided into two categories according to their mechanisms of action: inhibiting the generation of aqueous humor, and increasing the outflow of aqueous humor through the uveoscleral pathway. Glaucoma surgery also focuses only on creating paths to increase the rate of aqueous humor outflow. These methods are characterized by reducing intraocular pressure only but provide no direct treatment toward the pathological mechanism of aqueous humor outflow blockage in trabecular meshwork in glaucoma patients. Therefore, patients have to rely on using these treatments for life. In addition, as pathological changes in the trabecular meshwork continue to worsen, the efficacy of these treatments usually gradually declines after a few years. With the treatments fail to control intraocular pressure, glaucoma patients have to use a combination of drugs and surgical treatment.
2. The most commonly used surgical method for glaucoma is trabeculectomy. However, there are issues that affect the success rate of surgery. The most common issues include the postoperative fibrosis in Tenon's cyst in the filtration area, and abnormalities in the extracellular matrix proteins that cause scarring in the channel in the filtration area, leading to blockage of aqueous humor outflow and failure in lowering intraocular pressure. It has been shown that the main reasons for postoperative scarring of the filtration channel include proliferation, contraction and migration of fibroblasts in Tenon's cysts in the filtration area. In addition, various cytokines involve in and induce proliferation of the fibroblasts. Antimetabolic drugs such as mitomycin C (MMC) and 5-fluorouracil (5-FU), among others, are used to prevent scarring of the channel (bleb). However, antimetabolite may cause complications, such as postoperative filtration bleb leakage, corneal edema, conjunctival necrosis, scleral necrosis, and endophthalmitis. Therefore, drugs such as MMC and 5-FU are not optimal in preventing postoperative scarring of the filtration bleb.
3. Ultrasound emulsification removal of cataract in combination with artificial lens implant surgery has shown therapeutic efficacy in cataract patients; most patients show different degrees of vision improvement after treatment. However, part of the anterior lens capsule and the entire posterior lens capsule are retained in the surgery, leading to possible cell proliferation, migration and differentiation of the lens epithetic cells, which differentiate into fibroblasts. The fibroblasts eventually cause secondary cataract, which is a common postoperative complication of cataract surgeries. In this process, the balance between the transforming growth factor beta (TGF-beta) and the basic fibroblast growth factor (bFGF) is upset. The former (TGF-beta) regulates epithelial-mesenchymal transition of lens epithelial cells; deregulation of TGF-beta leads to abnormalities in the extracellular matrix proteins and capsular fibrosis. The latter (bFGF) regulates the regeneration and cell proliferation of the crystal structure. The disruption of equilibrium between TGF-beta and bFGF is the main cause of impaired vision in patients after cataract surgery. The occurrence of secondary cataracts may be lowered by selecting different designs of artificial lenses before surgery, or through Nd:YAG laser treatment of secondary cataracts. However, inflammation, elevated intraocular pressure, artificial lens damage, macular edema, retinal detachment, and other complications may still occur. Therefore, there remains a need for more effective treatment methods and preventive drugs for secondary cataract.
4. Proliferative vitreoretinopathy (PVR) is a common proliferation disorder in ophthalmology. Trauma, diabetes, retinal detachment and retinal detachment surgery can all cause proliferative disorders. The main pathological features of PVR are the proliferation of retinal pigment epithelial cells and glial cells, abnormal extracellular matrix proteins, and the formation of subretinal and preretinal fibrotic membrane. After the retina is damaged, retinal pigment epithelial cells (RPE), glial cells, macrophages, etc. migrate under the influence of cytokines and gather under the vitreous chamber and retina. During this process, the cells undergo phenotypic transition and form a fibrotic proliferative membrane capable of contraction, which eventually causes retinal detachment. At present, surgical removal of the proliferative membrane can significantly improve the affected vision, but there are still many patients with poor postoperative vision. In particular, severe PVR responds poorly to surgical methods, has unsatisfactory postoperative prognosis, and often requires multiple operations. In recent years, gene therapy of PVR has been a subject of study by many. However, technicalities of gene therapy have yet to be developed and optimized. Current gene therapy suffers from poor targeting efficacy and tissue specificity of the carrier, low success rate of transfection and safety issues, hindering its clinical application. Given the reasons above, postoperative ophthalmological proliferative diseases remain a major challenge in clinical ophthalmology. There has been much effort in finding safer and more effective drugs to prevent and treat proliferative disorders.

Salidroside is an important active ingredient extracted from the plant Rhodiola rosea (CAS: 10338-51-9, English name: Salidroside 2-(4-HYDROXYPHENYL) ETHYL-BETA-D-GLUCOPYRANOSIDE), with antioxidant, anti-inflammatory and protective against ischemia reperfusion injury activities, as well as relaxation of vascular smooth muscle and anti-fibrosis in liver, lung, and kidney.

The invention relates to use of salidroside or its derivatives in the inhibition of cell fibrosis in the pathogenesis and development of ophthalmological diseases, thereby preventing and treating ophthalmological diseases.

### BRIEF DESCRIPTION OF THE INVENTION

The technical problem to be solved by the present invention is the pharmaceutical application of salidroside or derivatives thereof as an inhibitor for treatment and prevention of diseases caused by abnormal extracellular matrix proteins, such as ophthalmological fibrosis; in particular, the application of salidroside or derivatives thereof in preparation of inhibitor drugs for prevention and treatment of trabecular meshwork fibrosis, and reduction of intraocular pressure in primary open angle glaucoma (POAG). The present application also explores the use of salidroside or derivatives thereof in other fibrosis-related ophthalmological disorders of extracellular matrix protein abnormality.

The purpose of the present invention is the application of salidroside or derivatives thereof in the preparation of inhibitor drugs for ophthalmological fibrosis disorders related to extracellular matrix protein abnormalities.

Furthermore, the ophthalmological fibrosis disorders related to extracellular matrix protein abnormalities described herein comprise one or more of glaucoma, conjunctiva scarring, pseudoptosis, skin scarring of the eye, age-related macular degeneration, diabetic retinopathy, or the secondary cataracts.

Furthermore, the ophthalmological fibrosis disorders related to extracellular matrix protein abnormalities described herein comprise surgical treatment failure caused by scarring of the filtration bleb after trabeculectomy, or scarring of the filtration bleb after trabeculectomy.

Additionally, another purpose of the present invention is developing an inhibitor pharmaceutical composition for treatment and prevention of ophthalmological fibrosis disorders related to extracellular matrix protein abnormalities, comprising an effective amount of salidroside as an active ingredient, in the form of tablets, capsules, pills, oral fluids, injectants, eye drops or eye ointments.

Furthermore, the pharmaceutical composition may comprise pharmaceutically acceptable excipient.

Furthermore, the injectant described herein may comprise an intravenous injectant, an intramuscular injectant, a subcutaneous injectant, a periocular injectant, a retrobulbar injectant, a subconjunctival injectant, a scleral injectant, or an intravitreal injectant. Furthermore, the pharmaceutical composition is an eye ointment, wherein the concentration of salidroside or derivatives thereof is 10 µM - 10 mM.

Furthermore, the pharmaceutical composition is an eye ointment, wherein the concentration of salidroside or derivatives thereof is 20 µM - 100 µM

Furthermore, the pharmaceutical composition is an eye drop, wherein the concentration of salidroside or derivatives thereof is 10 µM - 10 mM.

Furthermore, the pharmaceutical composition is an eye drop, wherein the concentration of salidroside or derivatives thereof is 20 µM - 100 µM.

Furthermore, the pharmaceutical composition is an intraocular injectant, wherein the concentration of salidroside or derivatives thereof is 20 µM - 100 µM

Furthermore, the pharmaceutical composition is a subconjunctival injectant, wherein the concentration of salidroside or derivatives thereof is 20 µM - 100 µM.

Furthermore, the pharmaceutical composition is a scleral injectant, wherein the concentration of salidroside or derivatives thereof is 20 µM - 100 µM.

Furthermore, the pharmaceutical composition is an intravitreal injectant wherein the concentration of salidroside or derivatives thereof is. 20 µM - 100 µM.

Furthermore, the pharmaceutical composition is an intracameral injectant, wherein the concentration of salidroside or derivatives thereof is. 20 µM - 100 µM.

The present application (1) explores the regulatory mechanism of salidroside in the accumulation of extracellular matrix proteins in normal trabecular meshwork induced by dexamethasone; (2) verifies the regulatory effect of salidroside on glaucoma-related factors, fibrous trabecular meshwork, and high intraocular pressure in mouse POAG model constructed by overexpression of TGF-beta2 with adenovirus vector.

In past experimental studies, salidroside has been shown to reduce tissue fibrosis in liver, lung, kidney and other organs. Latest research shows that salidroside also has anti-fibrotic effects. Salidroside inhibits fibrosis caused by TGF beta/smad signaling pathway in liver, lung, kidney and other organs. Our previous studies found that dexamethasone induces the expression of transforming growth factor beta (TGF beta), fibronectin (FN), collagen-IV (COL-IV), laminin (LN) and other proteins in cells, causing cell fibrosis. After intervention of salidroside, the expression level of TGF beta, FN, COL-IV and LN decreases (ibid).

Also provided is application of an inhibitor drug for treating and preventing ophthalmological fibrosis disorders related to extracellular matrix protein abnormalities. The present application explores the therapeutic effects of salidroside in other eye tissue fibrosis-related diseases such as glaucoma, conjunctiva scarring, pseudoptosis, eye skin scarring, age-related macular degeneration, diabetic retinopathy and other diseases related to abnormalities of extracellular matrix proteins as well as failure of surgery caused by filtration bleb scarring after trabeculectomy and the prevention of the scarring of the filtration bleb after trabeculectomy. The present application may provide new treatment for ophthalmological fibrosis.

The benefit of the present invention includes providing an application of an inhibitor drug for treating and preventing ophthalmological fibrosis disorders related to extracellular matrix protein abnormalities. The benefit of the present invention also includes inhibiting glaucoma and other related ophthalmological fibrosis disorders related to extracellular matrix protein abnormalities through anti-fibrotic effect achieved by inhibiting the expression of extracellular matrix proteins in the eye. Fibrosis may be effectively delayed and inhibited by the present invention using salidroside or derivatives thereof as an extracellular matrix protein inhibitor. Salidroside or derivatives thereof inhibit fibrosis significantly.

### DESCRIPTION OF THE DRAWINGS

FIG. 1 shows changes in FN expression after addition of salidroside.
FIG. 2 shows changes in COL-IV expression after addition of salidroside.
FIG. 3 shows changes in LN expression after addition of salidroside.
FIG. 4 shows changes in intraocular pressure after treatment with salidroside in mice with high intraocular pressure.
FIG. 5 shows changes in intraocular pressure after treatment with salidroside in mice with high intraocular pressure.

### DETAILED EMBODIMENTS

The exemplary embodiments of the present invention are further described below.

### Example 1.

The regulatory mechanism of salidroside on glaucoma-related factors and extracellular matrix level of trabecular meshwork is clarified.

### (1) Cell plating

Normal Trabecular Meshwork cells (NTM) were cultured with F12 containing 10% fetal bovine serum (FBS). When the normal human trabecular meshwork cells were 75%-80% confluent, cells were digested with pancreatic enzymes and planted into a 6-well plate with 2 mL culture media per well.

### (2) Experimental grouping

Normal human normal trabecular meshwork cells were used as a untreated control group. Normal human trabecular meshwork cells with dexamethasone added were used as the experimental control group. Normal human trabecular meshwork cells with dexamethasone and different concentrations of salidroside added were used as the experimental groups (The concentration of dexamethasone used in the experimental control group and the experimental groups was 10⁻⁷ mol/L. The concentrations of salidroside were 1, 3, 10, 30, 100 µmol/L, respectively). The trabecular meshwork cells were cultured for 0, 3, 6, 12, 24, 48 h.

### (3) Assay of trabecular meshwork extracellular matrix gene and protein expressions

Primer sequences for laminin (LN), collagen IV (Col-IV) and fibronectin (FN) mRNA were designed. Messenger RNA levels of LN, Col-IV, and FN were detected with qRT-PCR. Anti-LN, anti-Col-IV, and anti-FN antibodies were used to detect protein expression of the trabecular meshwork extracellular matrix protein in immunocytochemistry.

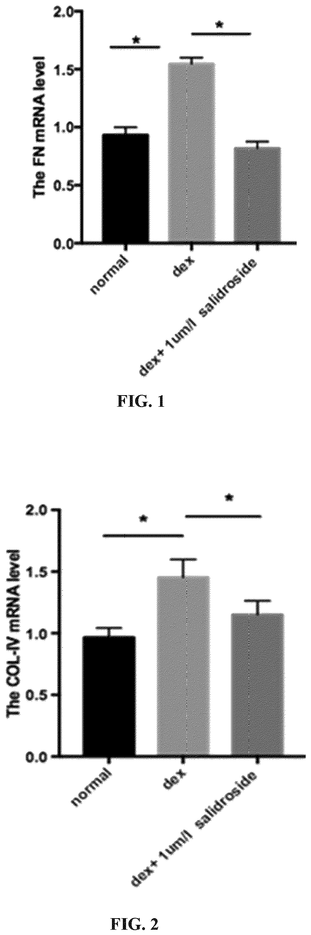

FIGs. 1-3 show the effect of salidroside detected by the qRT-PCR on the extracellular matrix proteins of human trabecular meshwork induced by the dexamethasone. It was found that dexamethasone increased the extracellular matrix proteins of the normal trabecular meshwork, and after addition of salidroside, the extracellular matrix proteins of the trabecular meshwork were reduced. Asterisks "*" in the figures indicate P < 0.05.

### Example 2.

1. Salidroside powder was dissolved in normal saline to make 40 mg/kg salidroside solution.
2. Male Balb/cJ mice 8 to 12 weeks old and weighted about 10 to 15 grams were used. The selected mice met the requirement of the first-level animal standards. Mice and feed were provided by the Experimental Animal Center of the Ninth People's Hospital, affiliated with Shanghai Jiaotong University School of Medicine. Vivarium temperature was controlled between 15 to 20 °C, and relative humidity was 55 to 75%, with 12h light. Food and drinking water were on an ad libitum basis. Mice that met the inclusion criteria were selected: (1) no eye disease; (2) pupillary light reflexes in both eyes in response to direct and indirect light were normal.
4. Intraocular pressure in both the left and the right eye of each conscious mouse was measured and recorded.
5. Weight measurements of each mouse were taken. 4% chloral hydrate was injected to anaesthetize mice at a dose of 10 µL/g body weight;
6. Mouse glaucoma model: 3 µL TGF-β2 overexpression adenovirus vector (3 × 10⁷ pfu/µL) was injected into the vitreous chamber to induce ocular hypertension. Empty adenovirus vector was injected into the contralateral eye as control.
7. After mice developed glaucoma, they were randomly divided into glaucoma control groups (n = 6), glaucoma + salidroside intervention group (n=10), glaucoma + salidroside treatment group (n=10) and normal control group (n=6), wherein glaucoma + salidroside intervention group received salidroside treatment immediately after adenovirus vector injections, glaucoma + salidroside treatment group received treatment immediately after intraocular pressure increased and stabilized. 40 mg/kg salidroside was injected into the peritoneal cavity once daily.
8. On the day of adenovirus vector intravitreal injection, mouse intraocular pressure was measured once. Intraocular pressure was measured again on the next day and subsequent selected days to avoid damage to the cornea caused by frequent measurement. In addition, intraperitoneal injections of salidroside took place after the measurement of intraocular pressure, and each measurement of intraocular pressure was carried out at the same time of the day to avoid circadian effect on intraocular pressure.

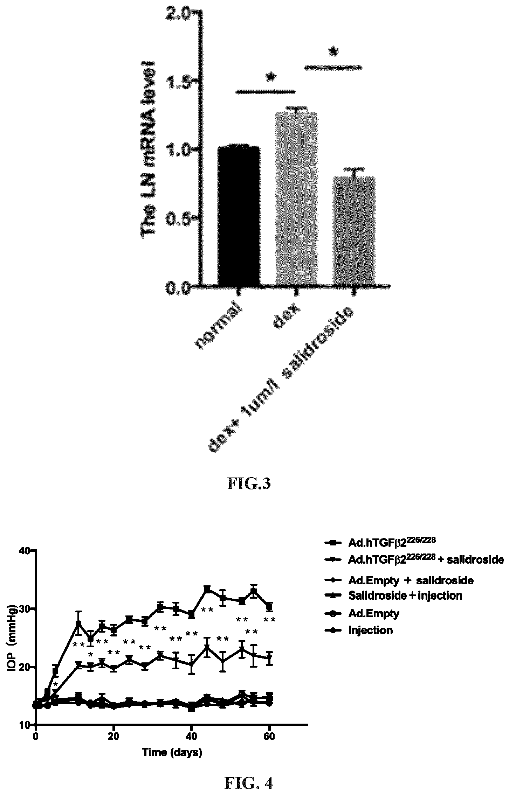

FIG. 4 shows that the intraocular pressure in mice in the salidroside intervention group was statistically significantly lowered than that in the glaucoma control group on the 11th day after the initiation of salidroside treatment.

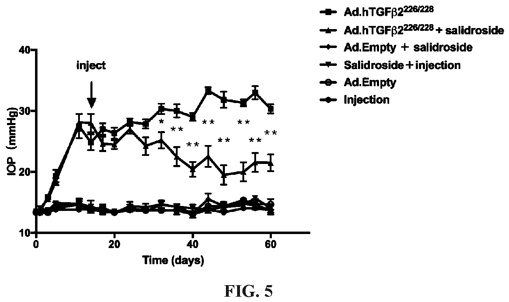

FIG. 5 shows that the intraocular pressure in mice in the glaucoma + salidroside treatment group was statistically significantly lowered than that in the glaucoma control group on the 18th day after the initiation of treatment of salidroside.

### Example 3.

1. Salidroside powder was dissolved in normal saline to prepare salidroside eye drops at the concentrations of 20 µM, 25 µM, 30 µM.
2. Fifty healthy, adult New Zealand white rabbit with body weight between 2.0 to 3.0 kg, consisting of equal numbers of male and female, were chosen for study. Rabbit glaucoma model was established by dosing the animals with 1% dexamethasone eye drops on both eyes 5 times a day for a total induction time of 3 weeks. Intraocular pressure was monitored starting from week 2. Glaucoma model was considered successfully established when intraocular pressure, measured at the same time of the day, was above 22 mmHg for a week. Glaucoma model was successful established on all 50 rabbits with the average intraocular pressure of 26.07 ± 2.03 mmHg (n = 100 eyes). Rabbits were randomly divided into 5 groups of 10: the normal control group, positive drug group (Travatan®, 0.004% travoprost eye drops), and three salidroside groups (20 µM, 25 µM, 30 µM salidroside).

Positive drug group was treated with Travatan® in the left eye, three times a day, two drops each time. Intraocular pressure was measured 4 hours after treatment. Treatment lasted for two weeks. The three salidroside groups were treated with salidroside eye drops three times a day, two drops each time. Intraocular pressure was measured 4 hours after treatment. Treatment lasted for two weeks. The normal control group received eye drops in both eyes three times a day, two drops each time. Intraocular pressure was measured 4 hours after receiving eye drops. Eye drops were administered for 2 weeks. One week after the start of experiment, the average pressure for each eye after each measurement in each group is shown in Table 1. It can be concluded from Table 1 that compared with the corresponding right eye as a control, the average intraocular pressure in the left eye measured from the positive drug group and the salidroside groups were all significant lowered (P < 0.01). The effects in the salidroside groups were better than that of the Travatan® group.

**Table 1 Average intraocular pressure.**

| Group | Left eye (mmHg) | Right eye (mmHg) | IOP reduction rate |
|---|---|---|---|
| Normal saline | 21.39 ± 1.09 | 21.75 ± 1.38 | 0% |
| Travatan® | 17.59 ± 1.06 | 22.10 ± 1.24 | 20.41% |
| 20 µM Salidroside | 15.57 ± 1.52 | 22.56 ± 1.72 | 30.98% |
| 25 µM Salidroside | 15.07 ± 1.19 | 23.03 ± 1.02 | 34.56% |
| 30 µM Salidroside | 14.37 ± 1.54 | 22.88 ± 1.09 | 37.19% |

The specific embodiments of the present invention are described in detail above, but only as exemplary embodiments, and the present invention is not limited to the specific embodiments described above. For those skilled in the art, any equivalent modification and substitution of the present invention are also within the scope of the present invention. Therefore, any equivalent transformation and modification made within the scope of the present invention is covered within the scope of the present invention.

## Claims

1. The application of salidroside or derivatives thereof in the preparation of pharmaceutical composition for the treatment of fibrotic ophthalmological disorders related to extracellular matrix protein abnormalities.

2. The application of claim **1,** wherein the fibrotic ophthalmological disorders related to extracellular matrix protein abnormalities described herein comprise one or more of glaucoma, conjunctiva scarring, pseudoptosis, skin scarring of the eye, age-related macular degeneration, diabetic retinopathy, or secondary cataracts.

3. The application of claim **1,** wherein the fibrotic ophthalmological disorders related to extracellular matrix protein abnormalities described herein comprise surgical treatment failure caused by scarring of the filtration bleb after trabeculectomy, or scarring of the filtration bleb after trabeculectomy.

4. A pharmaceutical composition for fibrotic ophthalmological disorders related to extracellular matrix protein abnormalities, comprising an effective amount of salidroside as an active ingredient, in the form of tablets, capsules, pills, oral fluids, injectants, eye drops or eye ointments.

5. The pharmaceutical composition of claim **4,** further comprises pharmaceutically acceptable excipients.

6. The pharmaceutical composition of claim **4,** wherein the pharmaceutical composition is an injectant comprising any one of an intravenous injectant, an intramuscular injectant, a subcutaneous injectant, a periocular injectant, a retrobulbar injectant, a subconjunctival injectant, a scleral injectant, an intravitreal injectant.

7. The pharmaceutical composition of claim **5,** wherein the pharmaceutical composition is in the form of an eye ointment, wherein the concentration of salidroside or derivatives thereof is 10 µM - 10 mM.

8. The pharmaceutical composition of claim **7,** wherein the pharmaceutical composition is in the form of an eye ointment, wherein the concentration of salidroside or derivatives thereof is 20 µM - 100 µM.

9. The pharmaceutical composition of claim **4,** wherein the pharmaceutical composition is in the form of an eye drop, wherein the concentration of salidroside or derivatives thereof is 10 µM - 10 mM.

10. The pharmaceutical composition of claim **9,** wherein the pharmaceutical composition is in the form of an eye drop, wherein the concentration of salidroside or derivatives thereof is 20 µM - 100 µM.

11. The pharmaceutical composition of claim **4,** wherein the pharmaceutical composition is in the form of an intraocular injectant, wherein the concentration of salidroside or derivatives thereof is 20 µM - 100 µM.

12. The pharmaceutical composition of claim **4,** wherein the pharmaceutical composition is in the form of a subconjunctival injectant, wherein the concentration of salidroside or derivatives thereof is 20 µM - 100 µM

13. The pharmaceutical composition of claim **4,** wherein the pharmaceutical composition is in the form of a subscleral injectant, wherein the concentration of salidroside or derivatives thereof is 20 µM - 100 µM.

14. The pharmaceutical composition of claim **11,** wherein the pharmaceutical composition is in the form of an intravitreal injectant wherein the concentration of salidroside or derivatives thereof is 20 µM - 100 µM.

15. The pharmaceutical composition of claim **11,** wherein the pharmaceutical composition is in the form of an intracameral injectant, wherein the concentration of salidroside or derivatives thereof is 20 µM - 100 µM
